# EUROPEAN PATENT APPLICATION

(11) **EP 1 777 213 A1**
(43) Date of publication of application: **25.04.2007**
(21) Application number: 06255353.2
(22) Date of filing: 18.10.2006
(51) Int. Cl.: C07C 215/28, C07C 269/04, C07C 271/16, C07D 301/24, C07D 303/36, C07D 301/26

(54) **Production method of aminochlorohydrin sulfate**

(30) Priority: 18.10.2005 JP 2005303673
(71) Applicant: Ajinomoto Co., Inc., Tokyo 104-8315 (JP)
(72) Inventor: Otake, Yasuyuki, Kawasaki-shi Kanagawa 210-8681 (JP); Hirose, Naoko, Kawasaki-shi Kanagawa 210-8681 (JP); Yatagai, Masanobu, Kawasaki-shi Kanagawa 210-8681 (JP)
(74) Representative: Nicholls, Kathryn Margaret

(57) **Abstract**

Highly pure (2R,3S)-3-tert-butoxycarbonylamino-1-chloro-2-hydroxy-4-phenylbutane or (2S,3R)-3-tert-butoxycarbonylamino-1-chloro-2-hydroxy-4-phenylbutane may be conveniently produced in high yield by: (a) reacting compound (1) with lithiumchloromethane to give compound (2) and at least a byproduct; (b) dissolving compound (2) and the byproduct in a polar solvent and adding water to the solution to precipitate compound (2) as crystals; (c) reducing the crystals of compound (2) to give compound (3) and at least its diastereomer as an impurity; (d) adding sulfuric acid thereto to give compound (4) and at least its diastereomer as an impurity; and (e) precipitating compound (4) as crystals from a solution containing acetic acid ester or acetic acid ester.

## Description

### FIELD OF THE INVENTION

The present invention relates to a particular aminochlorohydrin sulfate and crystal thereof, and production methods thereof. In addition, the present invention also relates to methods of producing an aminoepoxide from such an aminochlorohydrin sulfate.

### DISCUSSION OF THE BACKGROUND

3-Protected amino-1-chloro-2-hydroxy-4-phenylbutane represented by formula (6) (hereinafter to be also referred to as compound (6)) and 3-protected amino-1,2-epoxy-4-phenylbutane represented by formula (7) (hereinafter to be also referred to as compound (7)) are useful as intermediates for pharmaceutical compounds such as HIV protease inhibitors and the like. 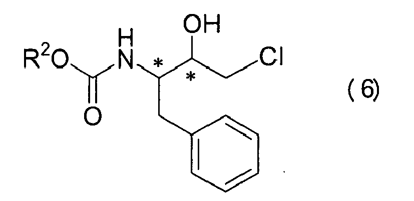 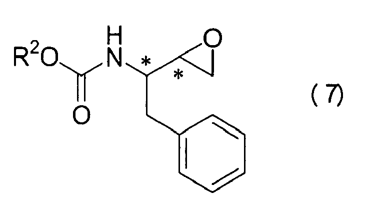 wherein R² is an alkyl group having 1 to 10 carbon atoms or an aralkyl group having 7 to 20 carbon atoms, * means an asymmetric carbon atom, and the configuration at the 2-position and the 3-position is (2R,3S) or (2S,3R).

The above-mentioned compound (7) can be obtained, for example, by treating the above-mentioned compound (6) with a base.

As production methods of the above-mentioned compound (6), several methods are known.

For example, (2R,3S)-3-tert-butoxycarbonylamino-1-halo-2-hydroxy-4-phenylbutane can be obtained by reducing (3S)-3-tert-butoxycarbonylamino-1-halo-2-oxo-4-phenylbutane with hydrogenated tri-tert-butoxy aluminumlithium in ether (*see,* P. Raddatz et al., J. Med. Chem., 1991, 34, 11, p. 3267; A. A. Malik et al., The 3rd International Conference on Organic Process Research & Development, Development of a Commercial Process for 2S,3S and 2R,3S-epoxides, 10-12th July 2000, Montreal; and T. Archibald et al., Scientific Update Conference Manual, Chiral USA '99, Full Scale Chiral Separations Using SMB, 4th May 1999, San Francisco, Scientific Update).

Moreover, for example, a method of producing the above-mentioned compound (6) by deprotecting 3-dibenzylamino-1-chloro-2-hydroxy-4-phenylbutane (hereinafter to be also referred to as compound (3)) by hydrogenation in the presence of an acid as necessary to give 3-amino-1-chloro-2-hydroxy-4-phenylbutane or an acidic salt thereof (hereinafter to be also referred to as compound (5)), and further protecting the amino group with a carbamate type protecting group is also known *(see,* P. Beaulieu et al., J. Org. Chem. 1996, 61, p. 3635). 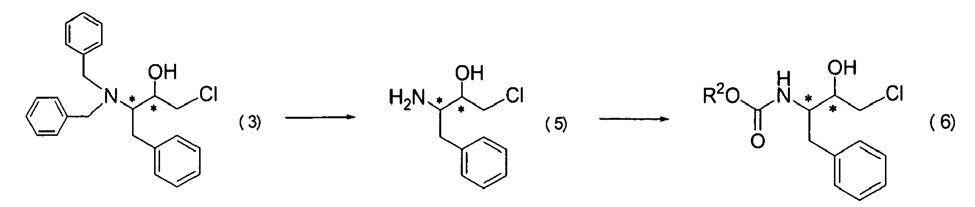 wherein R² is as defined above.

The above-mentioned compound (3) can be produced by a known method, for example, by halomethylating N,N-dibenzylphenylalanine ester (hereinafter to be also referred to as compound (1)) in the presence of lower alkyllithium and dihalomethane to give 3-dibenzylamino-1-chloro-2-oxo-4-phenylbutane (hereinafter to be also referred to as compound (2)), and reducing the compound *(see,* J. Barluenga et al, J. Org. Chem. 1995, 60, p. 6696).

However, with the above-mentioned production method, a byproduct occurs besides compound (2) in the halomethylation step of compound (1).

The present inventors found that the reaction needs to be carried out for a long time since the byproduct occurred in the above-mentioned halomethylation step deprotects the benzyl group of compound (3), markedly inhibiting the reaction to synthesize compound (5). To smoothly carry out the deprotection, therefore, compound (2) needs to be purified. In addition, when crude compound (3) is used, a problem of low purity of compounds (5), (6) and (7) also occurs.

In a step to produce compound (3) by reduction of compound (2), a diastereomer of compound (3) is further produced as impurity. For example, when (3S)-3-dibenzylamino-1-chloro-2-oxo-4-phenylbutane is reduced using sodium borohydride in methanol, a diastereomer, (2S,3S)-3-dibenzylamino-1-chloro-2-hydroxy-4-phenylbutane, is known to be produced in about 1 molar equivalent relative to 10-20 molar equivalents of the object (2R,3S)-form. Since it is difficult to obtain compound (3) as a crystal, removal of the diastereomer is also a huge task.

Various attempts have been heretofore made to remove the diastereomer.

In J. Barluenga et al, J. Org. Chem. 1995, 60, p. 6696, a purification method of compound (3) by silica gel column chromatography, which is unsuitable for industrial production, is employed.

In WO02/44136, a diastereomer is removed by dissolving compound (6) (N-carbamate-protected β-aminoalcohol) and the diastereomer as impurity in a polar organic solvent, adding water to the solution and performing crystal precipitation of the mixture, or by crystal precipitation of the N-protected-β-aminoalcohol from a diol or diol mixed solvent. However, since the method described in WO02/44136 precipitates a diastereomer, which is an impurity, as a solid, a certain level of diastereomer inevitably remains in the mother liquor along with the object compound, which problematically limits the purification level.

WO00/43357 discloses a production method of compound (7) (N-carbamate-protected β-aminoepoxide), wherein the N-carbamate-protected β-aminoalcohol containing the diastereomer as an impurity is dissolved in at least one kind of solvent selected from hydrocarbon solvents *etc.,* and an insoluble material is removed. However, in the method of WO00/43357, too, a diastereomer, (2S,3S)-form, is precipitated as crystals, and (2R,3S)-N-carbamate-protected β-aminoalcohol recovered in the mother liquor needs to be obtained by crystal precipitation. Consequently, the operation becomes complicated, and the object compound with high purity is difficult to obtain in a high yield, like the method described in WO02/44136.

Accordingly, a more convenient method of producing highly pure (2R,3S)-3-tert-butoxycarbonylamino-1-chloro-2-hydroxy-4-phenylbutane or (2S,3R)-3-tert-butoxycarbonylamino-1-chloro-2-hydroxy-4-phenylbutane in a high yield is desired.

### SUMMARY OF THE INVENTION

Accordingly, it is one object of the present invention to provide novel methods of producing (2R,3S)-3-tert-butoxycarbonylamino-1-chloro-2-hydroxy-4-phenylbutane or (2 S,3R)-3-tert-butoxycarbonylamino-1-chloro-2-hydroxy-4-phenylbutane.

It is another object of the present invention to provide novel methods of producing (2R,3S)-3-tert-butoxycarbonylamino-1-chloro-2-hydroxy-4-phenylbutane or (2S,3R)-3-tert-butoxycarbonylamino-1-chloro-2-hydroxy-4-phenylbutane, which are convenient and afford the product in high yield and high purity.

It is another object of the present invention to provide novel methods of producing (2R,3S)-3-tert-butoxycarbonylamino-1-chloro-2-hydroxy-4-phenylbutane or (2S,3R)-3-tert-butoxycarbonylamino-1-chloro-2-hydroxy-4-phenylbutane, which are industrially useful.

These and other objects, which will become apparent during the following detailed description, have been achieved by the inventors' discovery that compound (2) with a comparatively high purity can be obtained by reacting N,N-dibenzylphenylalanine ester with lithiumchloromethane to give compound (2) at least containing a byproduct, dissolving the compound in a polar solvent, adding water to the solution and producing crystals of compound (2) by crystal precipitation, and that the subsequent debenzylation reaction completes in a short time.

Furthermore, the present inventors have further found that compound (4) can be obtained as crystals in a high yield by reducing crystals of compound (2) to give compound (3) and at least its diastereomer as an impurity, adding sulfuric acid thereto to give compound (4) (which is a sulfate of compound (3)) and at least its diastereomer as an impurity, and performing crystal precipitation from a solution containing acetic acid ester or acetic acid ester, and that the diastereomer as an impurity has been fully removed from the crystal.

Accordingly, the present invention provides the following:
(1) A method of producing crystals of 3-dibenzylamino-1-chloro-2-hydroxy-4-phenylbutane sulfate represented by formula (4): 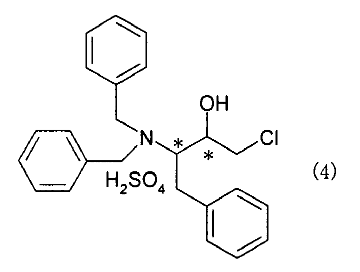 wherein * means an asymmetric carbon atom, and the configuration at the 2-position and the 3-position is (2R,3S) when the configuration of the following formula (3) is (2R,3S), or (2S,3R) when the configuration of the following formula (3) is (2S,3R),
   which method comprises:
   (a) reacting lithiumchloromethane with a N,N-dibenzylphenylalanine ester represented by formula (1): 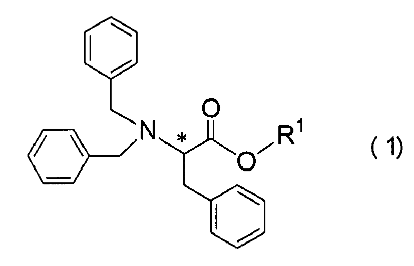 wherein R¹ is an alkyl group having 1 to 10 carbon atoms, an aryl group having 6 to 15 carbon atoms or an aralkyl group having 7 to 20 carbon atoms, each of which optionally having one or more substituents, or any of these groups containing one or more heteroatoms in a carbon skeleton, * means an asymmetric carbon atom, and the configuration at the 2-position is S or R, to obtain a 3-dibenzylamino-1-chloro-2-oxo-4-phenylbutane represented by formula (2): 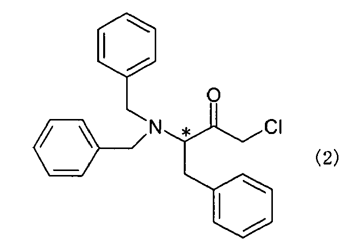 wherein * means an asymmetric carbon atom, the configuration at the 3-position is S when the configuration at the 2-position of the compound of formula (1) is S, or R when the configuration at the 2-position of the compound of formula (1) is R, and at least one byproduct;
   (b) dissolving said 3-dibenzylamino-1-chloro-2-oxo-4-phenylbutane represented by formula (2) and said byproduct in a polar solvent, and adding water to the solution to precipitate crystals of said 3-dibenzylamino-1-chloro-2-oxo-4-phenylbutane represented by formula (2);
   (c) reducing said crystals of 3-dibenzylamino-1-chloro-2-oxo-4-phenylbutane represented by formula (2) to obtain 3-dibenzylamino-1-chloro-2-hydroxy-4-phenylbutane represented by formula (3): 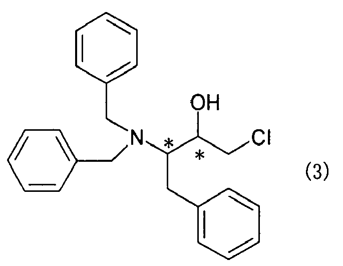 wherein * means an asymmetric carbon atom, the configuration at the 2-position and the 3-position is (2R,3S) when the configuration at the 3-position of the compound of formula (2) is S, or (2S,3R) when the configuration at the 3-position of the compound of formula (2) is R, and at least its diastereomer as an impurity;
   (d) adding sulfuric acid to the resulting 3-dibenzylamino-1-chloro-2-hydroxy-4-phenylbutane represented by formula (3) and at least its diastereomer as an impurity to obtain 3-dibenzylamino-1-chloro-2-hydroxy-4-phenylbutane sulfate represented by formula (4) and at least its diastereomer as an impurity; and
   (e) precipitating crystals of said 3-dibenzylamino-1-chloro-2-hydroxy-4-phenylbutane sulfate represented by formula (4) from a solution containing acetic acid ester or acetic acid ester.
(2) A method of producing a 3-protected amino-1-chloro-2-hydroxy-4-phenylbutane represented by formula (6): 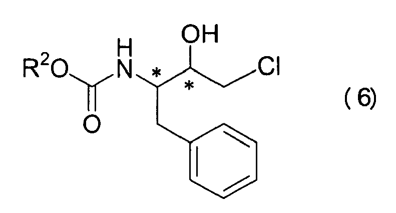 wherein R² is an alkyl group having 1 to 10 carbon atoms or an aralkyl group having 7 to 20 carbon atoms, * means an asymmetric carbon atom, the configuration at the 2-position and the 3-position is (2R,3S) when the configuration of the compound of formula (5) below is (2R,3S), or (2S,3R) when the configuration of the compound of formula (5) below is (2S,3R),
   which method comprises:
   (f) catalytically reducing crystals of 3-dibenzylamino-1-chloro-2-hydroxy-4-phenylbutane sulfate represented by formula (4), which are obtained according to the production method of the above-mentioned (1), to obtain 3-amino-1-chloro-2-hydroxy-4-phenylbutane sulfate represented by formula (5): 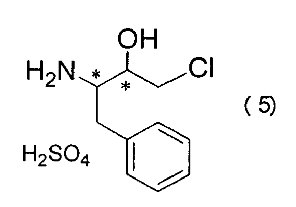 wherein * means an asymmetric carbon atom, the configuration at the 2-position and the 3-position is (2R,3S) when the configuration of the compound of formula (4) is (2R,3S), or (2S,3R) when the configuration of the compound of formula (4) is (2S,3R); and
   (g) protecting the amino group of said 3-amino-1-chloro-2-hydroxy-4-phenylbutane sulfate represented by formula (5) with a protecting agent under a neutral condition.
(3) A method of producing 3-protected amino-1,2-epoxy-4-phenylbutane represented by formula (7): 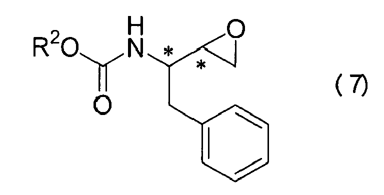 wherein R² is as defined in the above-mentioned (2), * means an asymmetric carbon atom, and the configuration at the 2-position and the 3-position is (2R,3S) when the configuration of the compound of formula (6) is (2R,3S), or (2S,3R) when the configuration of the compound of formula (6) is (2S,3R),
   which method comprises:
   (h) treating the 3-protected amino-1-chloro-2-hydroxy-4-phenylbutane represented by formula (6), which is obtained according to the production method of the above-mentioned (2), with a base.
(4) A method of producing crystals of 3-dibenzylamino-1-chloro-2-hydroxy-4-phenylbutane sulfate represented by formula (4): 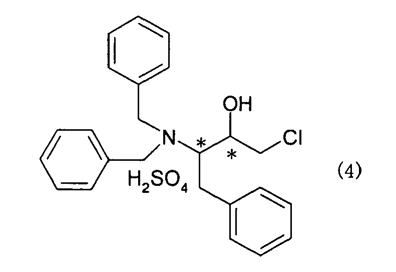 wherein * means an asymmetric carbon atom, the configuration at the 2-position and the 3-position is (2R,3S) when the configuration of the following formula (3) is (2R,3S), or (2S,3R) when the configuration of the following formula (3) is (2S,3R),
   which method comprises:
   (d) adding sulfuric acid to 3-dibenzylamino-1-chloro-2-hydroxy-4-phenylbutane represented by formula (3): 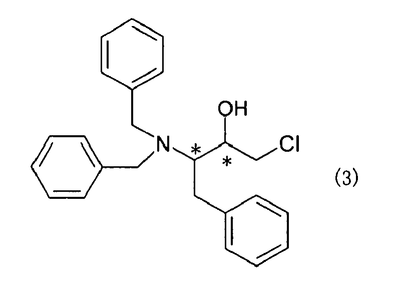 wherein * means an asymmetric carbon atom, and the configuration at the 2-position and the 3-position is (2R,3S) or (2S,3R), to obtain 3-dibenzylamino-1-chloro-2-hydroxy-4-phenylbutane sulfate represented by formula (4) and at least its diastereomer as an impurity; and
   (e) precipitating crystals of said 3-dibenzylamino-1-chloro-2-hydroxy-4-phenylbutane sulfate represented by formula (4) from a solution containing acetic acid ester or acetic acid ester.
(5) A method of producing crystals of (2R,3S)-3-dibenzylamino-1-chloro-2-hydroxy-4-phenylbutane sulfate represented by formula (4-a): 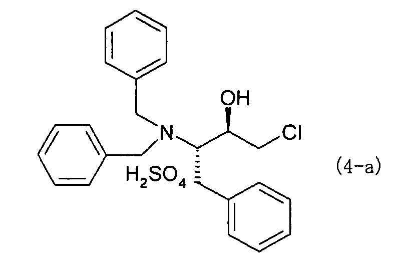 which comprises:
   (aa) reacting lithiumchloromethane with (S)-N,N-dibenzylphenylalanine ester represented by formula (1-a): 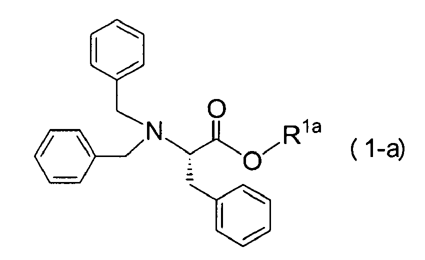 wherein R^{1a} is an alkyl group having 1 to 10 carbon atoms, an aryl group having 6 to 15 carbon atoms or an aralkyl group having 7 to 20 carbon atoms, each of which optionally having one or more substituents, or any of these groups containing one or more heteroatoms in a carbon skeleton, to obtain (3S)-3-dibenzylamino-1-chloro-2-oxo-4-phenylbutane represented by formula (2-a): 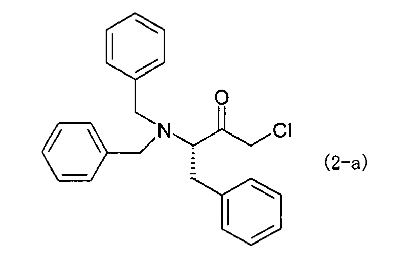 and at least one byproduct;
   (ab) dissolving said (3S)-3-dibenzylamino-1-chloro-2-oxo-4-phenylbutane represented by formula (2-a) and the byproduct in a polar solvent, and adding water to the solution to precipitate crystals of said (3S)-3-dibenzylamino-1-chloro-2-oxo-4-phenylbutane represented by formula (2-a);
   (ac) reducing said crystals of (3S)-3-dibenzylamino-1-chloro-2-oxo-4-phenylbutane represented by formula (2-a) to give (2R,3S)-3-dibenzylamino-1-chloro-2-hydroxy-4-phenylbutane represented by formula (3-a): 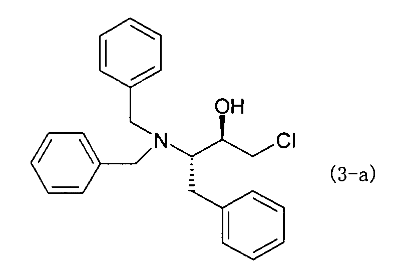 and at least its diastereomer as an impurity;
   (ad) adding sulfuric acid to said (2R,3S)-3-dibenzylamino-1-chloro-2-hydroxy-4-phenylbutane represented by formula (3-a) and at least its diastereomer as an impurity to give (2R,3S)-3-dibenzylamino-1-chloro-2-hydroxy-4-phenylbutane sulfate represented by formula (4-a) and at least its diastereomer as an impurity; and
   (ae) precipitating crystals of said (2R,3S)-3-dibenzylamino-1-chloro-2-hydroxy-4-phenylbutane sulfate represented by formula (4-a) from a solution containing acetic acid ester or acetic acid ester.
(6) A method of producing (2R,3S)-3-protected amino-1-chloro-2-hydroxy-4-phenylbutane represented by formula (6-a): 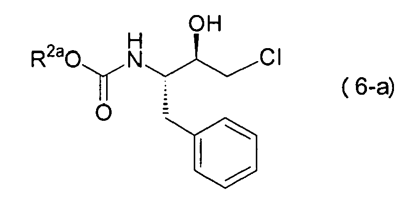 wherein R^{2a} is an alkyl group having 1 to 10 carbon atoms or an aralkyl group having 7 to 20 carbon atoms,
   which method comprises:
   (af) catalytically reducing crystals of said (2R,3S)-3-dibenzylamino-1-chloro-2-hydroxy-4-phenylbutane sulfate represented by formula (4-a), which is obtained according to the method of the above-mentioned (5), to obtain (2R,3S)-3-amino-1-chloro-2-hydroxy-4-phenylbutane sulfate represented by formula (5-a): 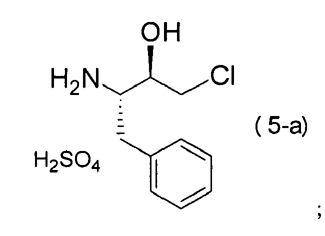 and
   (ag) protecting the amino group of said (2R,3S)-3-amino-1-chloro-2-hydroxy-4-phenylbutane sulfate represented by formula (5-a) with a protecting agent under a neutral condition.
(7) A method of producing (2R,3S)-3-protected amino-1,2-epoxy-4-phenylbutane represented by formula (7-a): 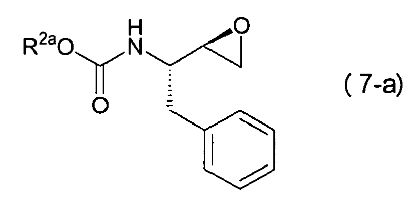 wherein R^{2a} is as defined in the above-mentioned (6),
   which method comprising:
   (ah) treating said (2R,3S)-3-protected amino-1-chloro-2-hydroxy-4-phenylbutane represented by formula (6-a), which is obtained according to the method of the above-mentioned (6), with a base.
(8) A method of producing crystals of (2S,3R)-3-dibenzylamino-1-chloro-2-hydroxy-4-phenylbutane sulfate represented by formula (4-b): 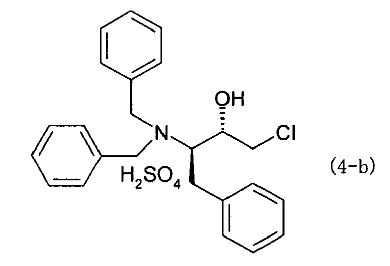 which method comprising:
   (ba) reacting lithiumchloromethane with (R)-N,N-dibenzylphenylalanine ester represented by formula (1-b): 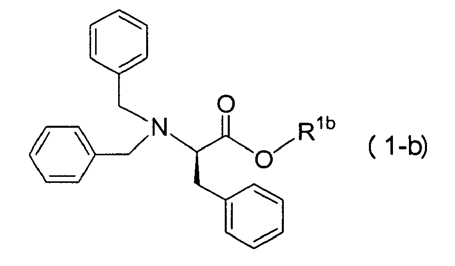 wherein R^{1b} is an alkyl group having 1 to 10 carbon atoms, an aryl group having 6 to 15 carbon atoms or an aralkyl group having 7 to 20 carbon atoms, each of which optionally having one or more substituents, or any of these groups containing one or more heteroatoms in a carbon skeleton, to obtain (3R)-3-dibenzylamino-1-chloro-2-oxo-4-phenylbutane represented by formula (2-b): 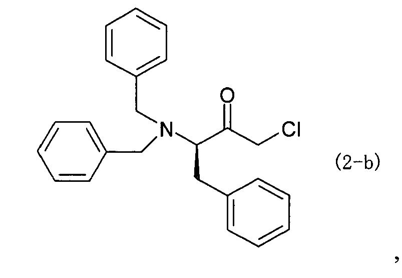 and at least a byproduct;
   (bb) dissolving said (3R)-3-dibenzylamino-1-chloro-2-oxo-4-phenylbutane represented by formula (2-b) and the byproduct, in a polar solvent and adding water to the solution to precipitate crystals of said (3R)-3-dibenzylamino-1-chloro-2-oxo-4-phenylbutane represented by formula (2-b);
   (bc) reducing the crystals of said (3R)-3-dibenzylamino-1-chloro-2-oxo-4-phenylbutane represented by formula (2-b) to give (2S,3R)-3-dibenzylamino-1-chloro-2-hydroxy-4-phenylbutane represented by formula (3-b): 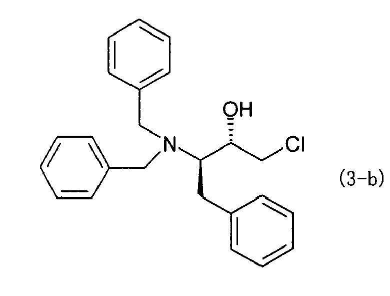 and at least its diastereomer as an impurity;
   (bd) adding sulfuric acid to said (2S,3R)-3-dibenzylamino-1-chloro-2-hydroxy-4-phenylbutane represented by formula (3-b) and at least its diastereomer as an impurity to give (2S,3R)-3-dibenzylamino-1-chloro-2-hydroxy-4-phenylbutane sulfate represented by formula (4-b) and at least its diastereomer as an impurity; and
   (be) precipitating crystals of said (2S,3R)-3-dibenzylamino-1-chloro-2-hydroxy-4-phenylbutane sulfate represented by formula (4-b) from a solution containing acetic acid ester or acetic acid ester.
(9) A method of producing (2S,3R)-3-protected amino-1-chloro-2-hydroxy-4-phenylbutane represented by formula (6-b): 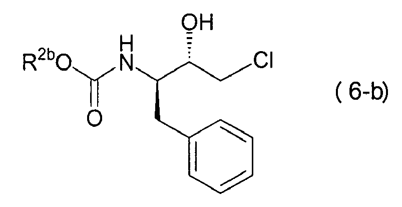 wherein R^{2b} is an alkyl group having 1 to 10 carbon atoms or an aralkyl group having 7 to 20 carbon atoms,
   which comprises:
   (bf) catalytically reducing crystals of said (2S,3R)-3-dibenzylamino-1-chloro-2-hydroxy-4-phenylbutane sulfate represented by formula (4-b), which is obtained according to method of the above-mentioned (8), to obtain (2S,3R)-3-amino-1-chloro-2-hydroxy-4-phenylbutane sulfate represented by formula (5-b): 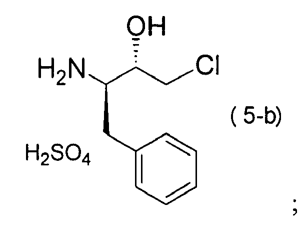 and
   (bg) protecting the amino group of said (2S,3R)-3-amino-1-chloro-2-hydroxy-4-phenylbutane sulfate represented by formula (5-b) with a protecting agent under a neutral condition.
(10) A method of producing (2S,3R)-3-protected amino-1,2-epoxy-4-phenylbutane represented by formula (7-b): 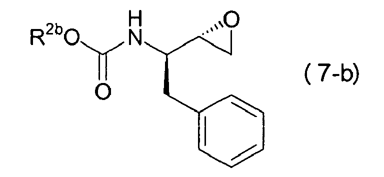 wherein R^{2a} is as defined in the above-mentioned (9),
   which comprises:
   (bh) treating said (2S,3R)-3-protected amino-1-chloro-2-hydroxy-4-phenylbutane represented by formula (6-b), which is obtained according to the method of the above-mentioned (9), with a base.
(11) The method of the above-mentioned (2) or (3), wherein R² is a tert-butyl group, a benzyl group, or a fluorenylmethyl group.
(12) The method of the above-mentioned (6) or (7), wherein R^{2a} is a tert-butyl group, a benzyl group, or a fluorenylmethyl group.
(13) The method of the above-mentioned (9) or (10), wherein R^{2b} is a tert-butyl group, a benzyl group, or a fluorenylmethyl group.
(14) The method of the above-mentioned (1), wherein R¹ is a methyl group, an ethyl group, an isobutyl group, or a benzyl group.
(15) The method of the above-mentioned (5), wherein R^{1a} is a methyl group, an ethyl group, an isobutyl group, or a benzyl group.
(16) The method of the above-mentioned (8), wherein R^{1b} is a methyl group, an ethyl group, an isobutyl group, or a benzyl group.
(17) The method of any one the above-mentioned (1)-(16), wherein the acetic acid ester is one or more kinds selected from the group consisting of methyl acetate, ethyl acetate, propyl acetate, and mixtures thereof.
(18) 3-Dibenzylamino-1-chloro-2-hydroxy-4-phenylbutane sulfate represented by formula (4): 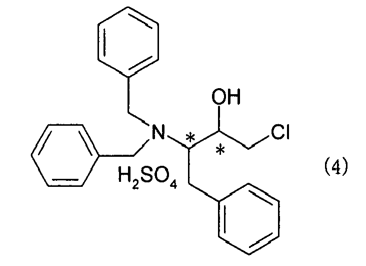 wherein * means an asymmetric carbon atom, and the configuration at the 2-position and the 3-position is (2R,3S) or (2S,3R).
(19) (2R,3S)-3-Dibenzylamino-1-chloro-2-hydroxy-4-phenylbutane sulfate represented by formula (4-a): 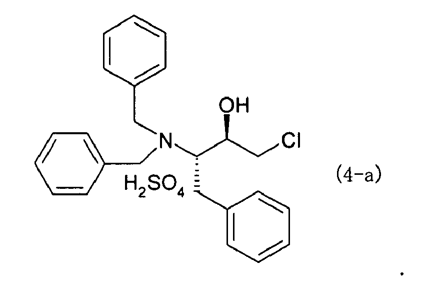
(20) (2S,3R)-3-Dibenzylamino-1-chloro-2-hydroxy-4-phenylbutane sulfate represented by formula (4-b):

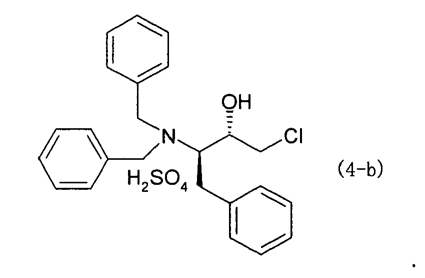

According to the producing method of the present invention, compounds of formulae (4), (5), (6) and (7) having a high purity can be obtained in a high yield by a more convenient method suitable for industrial production.

Particularly, according to the present invention, the byproduct resulting from the production of the compound of formula (2) can be removed efficiently and, as a result, the subsequent debenzylation reaction can be completed in a short time.

Moreover, the impurity, diastereomer, and other impurities and the like can be efficiently removed by converting the compound of formula (3) to its sulfate (the compound of formula (4)) and obtaining the compound of formula (4) as crystals. Therefore, the method of the present invention can conveniently afford (2R,3S)-3-dibenzylamino-1-chloro-2-hydroxy-4-phenylbutane or (2S,3R)-3-dibenzylamino-1-chloro-2-hydroxy-4-phenylbutane as crystals of sulfate having a high purity in a high yield.

For example, as in the method of the present invention, crystal precipitation of 3-dibenzylamino-1-chloro-2-hydroxy-4-phenylbutane as sulfate crystals from a solution containing acetic acid ester or acetic acid ester improves the ratio of (2R,3S)- or (2S,3R)-3-dibenzylamino-1-chloro-2-hydroxy-4-phenylbutane to its diastereomer to about 99:1, which may be further adjusted to 100:0.

According to the present invention, moreover, the compound of formula (5) obtained by catalytic reduction of crystals of the above-mentioned compound of formula (4), the compound of formula (6) obtained by protecting the amino group of the compound with a protecting agent under a neutral condition, and the compound of formula (7) obtained by treating the compound of formula (6) with a base can be obtained at high purity in a high yield.

### DETAILED DESCRIPTION OF THE PREFERRED EMBODIMENTS

The present invention is explained in more detail in the following.

In the present specification, for example, the expressions such as "3-dibenzylamino-1-chloro-2-hydroxy-4-phenylbutane sulfate represented by formula (4)," "N,N-dibenzylphenylalanine ester represented by formula (1)," and the like may be abbreviated simply as "compound (4)," "compound (1)," and the like. Moreover, in the following explanation in the present specification, when, for example, "compound (4)" is indicated, a comprehensive explanation of compound (4-a) and compound (4-b) is provided, unless otherwise specified.

### Explanation of R¹, R^{1a} and R^{1b}.

R¹ in the above-mentioned formula (1) is an alkyl group having 1 to 10 carbon atoms, an aryl group having 6 to 15 carbon atoms or an aralkyl group having 7 to 20 carbon atoms, each of which optionally having one or more substituents, or any of these groups containing one or more heteroatoms in a carbon skeleton.

As the above-mentioned "an alkyl group having 1 to 10 carbon atoms," for example, methyl group, ethyl group, propyl group, isopropyl group, n-butyl group, sec-butyl group, tert-butyl group, pentyl group, hexyl group, heptyl group, octyl group, nonyl group, decyl group and the like can be mentioned, preferably alkyl group having 1 to 6 carbon atoms (e.g., methyl group, ethyl group, propyl group, isopropyl group, n-butyl group, sec-butyl group, tert-butyl group, pentyl group, hexyl group, *etc.),* more preferably alkyl group having 1 to 4 carbon atoms (e.g., methyl group, ethyl group, propyl group, isopropyl group, n-butyl group, sec-butyl group, tert-butyl group, *etc.)* and the like can be mentioned.

As the above-mentioned "aryl group having 6 to 15 carbon atoms," preferred are phenyl group, naphthyl group, and the like, more preferred are phenyl group and the like.

As the above-mentioned "aralkyl group having 7 to 20 carbon atoms," preferred are benzyl group, phenethyl group, naphthylmethyl group, fluorenylmethyl group, and the like, more preferred are benzyl group, fluorenylmethyl group, and the like.

As the above-mentioned "any of these groups containing one or more heteroatoms in a carbon skeleton," for example, a group in which 1 to 3 carbon atoms in the carbon skeleton of the above-mentioned alkyl group having 1 to 10 carbon atoms, an aryl group having 6 to 15 carbon atoms or an aralkyl group having 7 to 20 carbon atoms are substituted by a heteroatom such as nitrogen, oxygen, sulfur atom, and the like can be mentioned. As preferable "group containing one or more heteroatoms in a carbon skeleton of any of these groups," for example, methylthioethyl group, t-butylthiomethyl group, tritylthiomethyl group, (p-methylbenzyl)thiomethyl group, (p-methoxybenzyl)thiomethyl group, t-butoxymethyl group, benzyloxymethyl group, t-butoxyethyl group, benzyloxyethyl group, 4-(t-butoxy)phenylmethyl group, 4-benzyloxyphenylmethyl group, phenylthiomethyl group, and the like can be mentioned.

The "substituent" that the "alkyl group having 1 to 10 carbon atoms, an aryl group having 6 to 15 carbon atoms or an aralkyl group having 7 to 20 carbon atoms, or any of these groups containing one or more heteroatoms in a carbon skeleton" as the above-mentioned R¹ may have, is not particularly limited as long as the substituent does not have an adverse influence on the reaction of the present invention and, for example, alkoxy group (preferably alkoxy group having 1 to 7 carbon atoms), nitro group, alkyl group (preferably alkyl group having 1 to 6 carbon atoms), halogen atom, and the like can be mentioned.

As the above-mentioned R¹, preferred are, for example, methyl group, ethyl group, isopropyl group, benzyl group, and the like, more preferred are methyl group, ethyl group, benzyl group, and the like.

As R^{1b} in the above-mentioned formula (1-a) and R^{1a} in the above-mentioned formula (1-b), those similar to the above-mentioned R¹ can be mentioned.

### Explanation of R², R^{2a} and R^{2b}

In the above-mentioned formulas (6) and (7), R² is an alkyl group having 1 to 10 carbon atoms or aralkyl having 7 to 20 carbon atoms. As the above-mentioned "alkyl group having 1 to 10 carbon atoms," for example, methyl group, ethyl group, propyl group, isopropyl group, n-butyl group, sec-butyl group, tert-butyl group, pentyl group, hexyl group, heptyl group, octyl group, nonyl group, decyl group, and the like can be mentioned. Preferred are alkyl group having 1 to 6 carbon atoms (e.g., methyl group, ethyl group, propyl group, isopropyl group, n-butyl group, sec-butyl group, tert-butyl group, pentyl group, hexyl group, *etc.),* more preferred are alkyl group having 1 to 4 carbon atoms (e.g., methyl group, ethyl group, propyl group, isopropyl group, n-butyl group, sec-butyl group, tert-butyl group, *etc.*), and the like.

As the above-mentioned "aralkyl group having 7 to 20 carbon atoms," preferred are benzyl group, phenethyl group, naphthylmethyl group, fluorenylmethyl group, and the like, more preferred are benzyl group, fluorenylmethyl group, and the like.

As the above-mentioned R², preferred are, for example, tert-butyl group, benzyl group, fluorenylmethyl group, and the like, more preferably tert-butyl group, benzyl group, and the like.

As R^{2a} in the above-mentioned formulas (6-a) and (7-a) and as R^{2b} in formulas (6-b) and (7-b), those similar to the above-mentioned R² can be mentioned.

Each step in the production method of the present invention is explained in detail in the following.

### Explanation of step (a).

In step (a), N,N-dibenzylphenylalanine ester represented by the above-mentioned formula (1) is reacted with lithiumchloromethane to give the above-mentioned 3-dibenzylamino-1-chloro-2-oxo-4-phenylbutane represented by formula (2), which contains at least a byproduct.

The reaction of step (a) is known and, for example, the method described in J. Barluenga et al., J. Org. Chem., 1995, 60, p. 6698 can be referred to.

Lithiumchloromethane to be used in step (a) is, for example, formed in a reaction system by previously adding bromochloromethane or iodochloromethane to the reaction system and then adding alkyllithium. As preferable alkyllithium, for example, alkyllithium having 1 to 6 carbon atoms such as n-butyllithium, methyllithium, and the like can be mentioned, and n-butyllithium is particularly preferable.

The reaction of step (a) is preferably carried out in the presence of a solvent. As preferable reaction solvents, for example, ether solvents such as tetrahydrofuran, diethyl ether, methyl tert-butyl ether, and the like, mixed solvents of these ether solvents, nonpolar solvents such as benzene, toluene, hexane, heptane, and the like, and the like can be mentioned. Tetrahydrofuran, a mixed solvent of tetrahydrofuran and toluene or heptane, and the like are particularly preferable.

Preferable reaction temperature of step (a) is -50°C to -100°C, particularly preferably -70°C to -100°C.
In the reaction of step (a), a byproduct is produced *(see, e.g.,* J. Barluenga et al., J. Org. Chem., 1997, 62, p. 5974; G. Köbrich et al., Tetrahedron, 1968, 24, p. 4343 and the like). Such byproducts are removed in step (b) to be explained next.

Since the above-mentioned step (aa) and step (ba) are similar to step (a), explanation of these steps is omitted.

### Explanation of step (b).

In step (b), crystals of compound (2) are precipitated by dissolving the above-mentioned compound (2) and the byproduct in a polar solvent, and adding water to the solution.

In step (b), the above-mentioned compound (2) and the byproduct are dissolved in a polar solvent. As the polar solvent, organic solvents miscible with water, for example, methanol, ethanol, isopropanol, acetone, 2-butanone, acetonitrile, tetrahydrofuran, and mixed solvents of any of these solvents can be mentioned, with particular preference given to methanol. The polar solvent may contain other solvents besides polar solvent to the extent that the effect of the invention is not inhibited.

While the temperature during dissolution of the above-mentioned compound (2) and at least a byproduct in a polar solvent is not particularly limited, it is preferably -20°C to 60°C, more preferably -10°C to 30°C. While the amount of the polar solvent to be used is not particularly limited, either, for example, 2 ml to 20 ml of a solvent is preferably used per 1 g of the above-mentioned compound (2).

Then, water is added to the above-mentioned solution to allow precipitation of crystals of the above-mentioned compound (2).

While the amount of water to be added is not particularly limited, it is preferably 1% to 100%, more preferably 5% to 50%, in a weight ratio relative to the above-mentioned polar solvent.

The temperature for crystal precipitation is preferably -20°C to 60°C, particularly preferably -20°C to 30°C. While the temperature of water to be added is not particularly limited, it is preferably the same as the temperature of crystal precipitation. While the manner of addition of water is not particularly limited, it is preferably added gradually over 30 minutes to 4 hours.

The above-mentioned crystal precipitation may be performed with stirring or under still standing. Where necessary, a seed crystal of the crystal of object compound (2) is added before, during or after addition of water, whereby crystallization is performed more easily. In addition, where necessary, crystal precipitation may be performed by cooling the crystal - solvent system during or after addition of water.

In step (b), the object compound (compound (2)) is obtained as crystals, the impurity is made to remain in the mother liquor and removed. In step (b), therefore, compound (2) can be efficiently obtained with a high purity.

The crystal of the obtained compound (2) may be further washed with a solvent such as water, methanol, ethanol, acetone, heptane, hexane, toluene, and the like.

The crystal of compound (2) is subjected to step (c) to be explained below.

Since the above-mentioned step (ab) and step (bb) are similar to step (b), explanation of these steps is omitted.

### Explanation of step (c).

In step (c), the crystal of the above-mentioned compound (2) obtained in step (b) by crystal precipitation is reduced to give compound (3) and at least its diastereomer as an impurity.

While the reduction in step (c) may be carried out by any method, it is preferably carried out in the presence of a reducing agent and a solvent.

As a reducing agent preferable for the reduction in step (c), for example, sodium borohydride, lithium aluminum hydride, and the like can be mentioned.

A solvent preferable for the reduction in step (c) varies depending on the kind of the reducing agent. As a preferable solvent when sodium borohydride is used as a reducing agent, a protic solvent such as methanol, ethanol, isopropanol, and the like; a mixed solvent of any of these protic solvents; and an aprotic solvent such as tetrahydrofuran, dichloromethane, methyl acetate, ethyl acetate, isopropyl acetate, diethyl ether, methyl tert-butyl ether, hexane, heptane, toluene, and the like; and the like can be mentioned. Preferred is a protic solvent such as methanol, ethanol, and the like, a mixed solvent of any of these protic solvents, and an aprotic solvent such as dichloromethane, ethyl acetate, isopropyl acetate, tetrahydrofuran, and the like, and the like. As a preferable solvent when lithium aluminum hydride is used as a reducing agent, an aprotic solvent such as tetrahydrofuran, dichloromethane, diethyl ether, methyl tert-butyl ether, toluene, and the like, a mixed solvent thereof, and the like can be mentioned.

The reaction temperature of the reduction in step (c) is generally -100°C to 50°C, preferably -100°C to 10°C.

After completion of the reduction reaction, the reaction is quenched with an acid. As the acid to be used for quenching the reaction, for example, hydrochloric acid, sulfuric acid, phosphoric acid, acetic acid, citric acid, and the like can be mentioned, with preference given to hydrochloric acid, sulfuric acid, phosphoric acid, and the like.

While the temperature at which to quench the reduction reaction is not particularly limited, it is preferably the same as the temperature of the reduction reaction.

After quenching the reduction reaction, the above-mentioned compound (3) is extracted with water and an organic solvent. As the organic solvent to be used for the extraction, dichloromethane, chloroform, ethyl acetate, isopropyl acetate, toluene, diethyl ether, methyl tert-butyl ether, and the like are preferable, and dichloromethane, ethyl acetate, isopropyl acetate, and the like are particularly preferable. The organic solvent may contain other solvent to the extent that the effect of the present invention is not inhibited.

While an embodiment wherein a reduction reaction is quenched, and then the above-mentioned object compound (3) is extracted has been given here, extraction may be performed by evaporating the reaction solvent after completion of the reaction, and substituting the solvent with the above-mentioned organic solvent.

While the amounts of the organic solvent and water to be used for the above-mentioned extraction are not particularly limited, 2 ml to 20 ml of each of them is preferably used per 1 g of the above-mentioned compound (3). While the extraction temperature is not particularly limited, either, it is preferably -10°C to 40°C, particularly preferably 10°C to 30°C.

In the above-mentioned reduction, it is known that the product ratio of compound (3) and its impurity (diastereomer) varies depending on the kind of the reducing agent and reaction conditions, where production of the diastereomer can be inhibited to a certain degree by selecting suitable reduction conditions *(e.g., see* J. Barluenga et al., J. Org. Chem., 1995, 60, p. 6696). However, inhibition of the production of the diastereomer based solely on the selection of reduction conditions in this step is insufficient. Accordingly, in the method of the present invention, the diastereomer is removed by subjecting compound (3) and at least its diastereomer as an impurity, which is obtained in this step, to step (d) and step (e) explained below.

Since the above-mentioned step (ac) and step (bc) are similar to step (c), explanation of these steps is omitted.

### Explanation of step (d).

In step (d), compound (4) and at least its diastereomer as an impurity are obtained by adding sulfuric acid to the above-mentioned compound (3) and at least its diastereomer as an impurity. Compound (4) is a sulfate of compound (3), and first obtained by the present inventors.

The reaction of step (d) is preferably carried out in a solvent. As a preferable solvent, for example, acetic acid ester [e.g., methyl acetate, ethyl acetate, propyl acetate (e.g., isopropyl acetate)], dichloromethane, methanol, ethanol, tetrahydrofuran, acetonitrile, isopropanol, and the like can be mentioned. More preferred are methyl acetate, ethyl acetate, isopropyl acetate, dichloromethane, and the like. Alternatively, an organic solvent layer containing compound (3) and at least its diastereomer as an impurity, which are obtained by extracting the reaction system after quenching the reduction reaction in the above-mentioned step (c) with water and the organic solvent may be directly subjected to step (d).

The amount of sulfuric acid to be used in step (d) is preferably 0.5 molar equivalent to 2 molar equivalents, particularly 0.9 molar equivalent to 1.2 mol equivalents, relative to the above-mentioned compound (3) and at least its diastereomer as an impurity.

While the temperature of sulfuric acid to be added is not particularly limited, it is preferably -10°C to 40°C, particularly preferably -10°C to 30°C.

Compound (4) and at least its diastereomer as an impurity, which are obtained in step (d), are subjected to the next step (e).

Since the above-mentioned step (ad) and step (bd) are similar to step (d), explanation of these steps is omitted.

### Explanation of step (e).

In step (e), crystals of compound (4) and at least its diastereomer as an impurity, which are obtained in the above-mentioned step (d), are subjected to precipitation from acetic acid ester or a solution containing acetic acid ester to give crystals of compound (4). The crystal of compound (4) has also been first found by the present inventors.

As acetic acid ester to be used for crystal precipitation in step (e), for example, methyl acetate, ethyl acetate, propyl acetate (*e.g.,* isopropyl acetate), and the like can be mentioned, and ethyl acetate is particularly preferable.

As the above-mentioned solution containing acetic acid ester, for example, a mixed solvent of the above-mentioned acetic acid ester and a solvent such as dichloromethane, methanol, ethanol, isopropanol, acetonitrile, tetrahydrofuran, and the like can be mentioned, and a mixed solvent of ethyl acetate and dichloromethane, and a mixed solvent of isopropyl acetate and dichloromethane are particularly preferable.

While the amount of the acetic acid ester to be used for the above-mentioned solution containing acetic acid ester or acetic acid ester is not particularly limited, it is preferably 1 ml to 30 ml per 1 g of the above-mentioned compound (4).

The temperature of crystal precipitation is preferably -20°C to 60°C, particularly preferably 0°C to 40°C.

When the above-mentioned acetic acid ester or a solution containing acetic acid ester is added to the above-mentioned compound (4) and at least its diastereomer as an impurity, the manner of addition is not particularly limited. However, gradual addition over 30 minutes to 4 hours is preferable. In addition, while the temperature of the above-mentioned acetic acid ester or a solution containing acetic acid ester is not particularly limited, it is preferably the same as the temperature of crystal precipitation.

The crystal precipitation in step (e) may be performed with stirring or under standing still. Where necessary, a seed crystal of the crystal of the object compound (4) may be added to more easily perform crystallization.

During crystal precipitation in step (e), not only the diastereomer, which is an impurity, but also other impurities can be simultaneously removed by remainig in the mother liquor. Thus, crystals of the above-mentioned compound (4) can be efficiently obtained at high purity.

Where necessary, the crystal of the obtained compound (4) may be washed with a solvent such as acetic acid ester (*e.g.,* methyl acetate, ethyl acetate, propyl acetate (e.g., isopropyl acetate)), diethyl ether, methyl tert-butyl ether, tetrahydrofuran, water, methanol, ethanol, acetone, heptane, hexane, toluene, and the like.

The crystal of compound (4) is used for the synthesis of compound (5) to be explained below.

Since the above-mentioned step (ae) and step (be) are similar to step (e), explanation of these steps is omitted.

### Explanation of step (f).

In step (f), the crystal of compound (4) obtained in the above-mentioned step (e) is subjected to catalytic reduction to give 3-amino-1-chloro-2-hydroxy-4-phenylbutane sulfate represented by formula (5).

The above-mentioned catalytic reduction can be carried out by hydrogenation preferably in a solvent in the presence of a catalyst.

The catalyst to be used for the above-mentioned catalytic reduction is, for example, palladium carbon, palladium carbon hydroxide, Lindler's catalyst, and the like, and palladium carbon, palladium carbon hydroxide, and the like are preferable.

As the solvent to be used for the above-mentioned catalytic reduction, for example, methanol, ethanol, isopropanol, a mixed solution of any of these solvents and water, and the like can be mentioned, and methanol, ethanol, and the like are preferable.

Preferable reaction temperature of the above-mentioned catalytic reduction is 0°C to 70°C, particularly preferably 10°C to 50°C.

In step (f), since the crystal of the starting substance (compound (4)) is a sulfate, compound (5) obtained by catalytic reduction is also a sulfate. Thus, in step (f), compound (5) is highly stable in the reaction system and its yield can also be advantageously increased.

Since the above-mentioned step (af) and step (bf) are similar to step (f), explanation of these steps is omitted.

### Explanation of step (g).

In step (g), compound (6) is obtained by protecting the amino group of compound (5) under a neutral condition using a protecting agent.

As the protection agent usable for step (g), for example, a protection agent (e.g., di-tert-butylcarbonate, *etc.)* capable of introducing a tert-butoxycarbonyl group, and the like can be mentioned.

The above-mentioned reaction needs to be carried out under a neutral condition, since the above-mentioned protection agent is unstable to acids. To neutralize this reaction system, compound (5), which is a sulfate, is neutralized. For example, the reaction system is neutralized with a base (e.g., sodium hydroxide, sodium carbonate, *etc.),* and then the above-mentioned protection agent is added to the neutralized reaction system.

The above-mentioned reaction is preferably carried out, for example, in a solvent such as dichloromethane, ethyl acetate and the like.

Preferable reaction temperature of the above-mentioned reaction is 10°C to 50°C, particularly preferably 20°C to 40°C.

Preferable reaction time of the above-mentioned reaction is 0.5 hour to 24 hours, particularly preferably 1 hour to 12 hours.

Where necessary, the obtained compound (6) may be further purified by a method such as recrystallization and the like.

Since the above-mentioned step (ag) and step (bg) are similar to step (g), explanation of these steps is omitted.

### Explanation of step (h).

In step (h), compound (7) is obtained by treating compound (6) with a base.

This base treatment reaction is known and, for example, P. L. Beaulieu et al., Tetrahedron Lett., 1995, 36, p. 3317 and the like may be referred to for carrying out the reaction.

Since the above-mentioned step (ah) and step (bh) are similar to step (h), explanation of these steps is omitted.

Other features of the invention will become apparent in the course of the following descriptions of exemplary embodiments which are given for illustration of the invention and are not intended to be limiting thereof.

### EXAMPLES

In the following examples, the ratios of the object compounds described in the Examples and their diastereomers are molar ratios.

### Example 1. Production of crystal of (2R,3S)-3-dibenzylamino-1-chloro-2-hydroxy-4-phenylbutane sulfate.

### Step (1a): Production of (3S)-3-dibenzylamino-1-chloro-2-oxo-4-phenylbutane (containing byproduct).

To (2S)-N,N-dibenzyl-L-phenylalanine benzyl ester (3.4 g, net. 3.0 g, 6.89 mmol, 88.2 wt%) were added hexane (10.6 ml), tetrahydrofuran (11.3 ml), and bromochloromethane (1.23 ml), and the mixture was cooled to -70°C under argon atmosphere with stirring. While stirring the solution, 2.64 M solution of n-butyllithium in hexane (5.35 ml) was added dropwise at the same temperature over 2 hours. After stirring the reaction mixture at the same temperature for 30 minutes, the reaction mixture was poured into 4 M aqueous hydrochloric acid solution (3.53 ml) at once to quench the reaction. The reaction mixture was allowed to stand still and the organic layer was separated. As a result of HPLC analysis, it was found that (3S)-3-dibenzylamino-1-chloro-2-oxo-4-phenylbutane (1.94 g, yield 74.5%) of the object compound was contained in the organic layer, and the peak area ratio was 57.4%. In addition, the total peak area ratio of impurity was 42.6%.

### Step (1b): Production of crystal of (3S)-3-dibenzylamino-1-chloro-2-oxo-4-phenylbutane.

Under reduced pressure, the solvent was evaporated from the organic layer (containing (3S)-3-dibenzylamino-1-chloro-2-oxo-4-phenylbutane (1.94 g, 5.13 mmol)) obtained in the above-mentioned step (1a), methanol (15 ml) was added to the residue, and the mixture was cooled to 10°C. To this solution was added a crystal (10 mg) of (3S)-3-dibenzylamino-1-chloro-2-oxo-4-phenylbutane as a seed crystal, and the mixture was further stirred for 1 hour. After cooling the obtained slurry to -10°C, water (2.3 ml) was added, and the mixture was further stirred for 2 hours. The obtained slurry was filtered, and the crystal was washed with a mixed solvent of methanol (7.2 ml) and water (1.8 ml). The obtained crystals were dried under reduced pressure to give (3S)-3-dibenzylamino-1-chloro-2-oxo-4-phenylbutane as crystals (1.68 g, 4.45 mmol, yield 86.6%). As a result of HPLC analysis, it was found that the peak area ratio of the compound was 92.4%.
melting point 45.8 to 46.8°C
¹H-NMR (CDCl₃, 400 MHz); δppm 2.99 (dd, J=9.7, 3.6Hz, 1H), 3.20-3.26 (m, 1H), 3.58 (d, J=13.6Hz, 2H), 3.65-3.69 (m, 1H), 3.81-3.85 (m, 3H), 4.41-4.43 (m, 1H), 7.13-7.37 (m, 15H)
¹³C-NMR (CDCl₃, 100 MHz); δppm 29.4, 48.3, 55.1, 67.1, 126.7, 128.0, 129.0, 129.1, 129.4, 129.9, 138.9, 139.1, 201.0
mass spectrum; m/z = 378.71 (M+H)⁺
[α]_{D}²⁰= -110.3°C (c = 1.0, methanol)

### Step (1c): Production of (2R,3S)-3-dibenzylamino-1-chloro-2-hydroxy-4-phenylbutane (containing (2S,3S)-form thereof as diastereomer).

The crystal (5.1 g, 13.5 mmol) of (3S)-3-dibenzylamino-1-chloro-2-oxo-4-phenylbutane obtained in the above-mentioned step (1b) was dissolved in a mixed solvent of methanol (41 ml) and dichloromethane (15.3 ml) under argon atmosphere, and the mixture was cooled to -10°C. To this solution was added, over 10 min, sodium borohydride (255 mg) divided in 10 portions, and the mixture was stirred for 1 hour. 36% Concentrated hydrochloric acid (0.579 ml) was added to the reaction solution to quench the reaction. Dichloromethane (10.2 ml) and water (25.5 ml) were added at 20°C to partition the solution and the organic layer was washed with water (25.5 ml) and saturated brine again to give an organic layer containing (2R,3S)-3-dibenzylamino-1-chloro-2-hydroxy-4-phenylbutane and its diastereomer (2S,3S)-form as an impurity.

### Step (1d): Production of (2R,3S)-3-dibenzylamino-1-chloro-2-hydroxy-4-phenylbutane sulfate.

To the organic layer obtained in the above-mentioned step (1c) was added sulfuric acid (0.718 ml) at 20°C to give an organic layer containing (2R,3S)-3-dibenzylamino-1-chloro-2-hydroxy-4-phenylbutane sulfate and its diastereomer ((2S,3S)-form) as an impurity.

### Step (1e): Production of crystal of (2R,3S)-3-dibenzylamino-1-chloro-2-hydroxy-4-phenylbutane sulfate.

To the organic layer obtained in the above-mentioned step (1d) was added ethyl acetate (10.2 ml), further added a seed crystal (10 mg) of (2R,3S)-3-dibenzylamino-1-chloro-2-hydroxy-4-phenylbutane sulfate, and the mixture was stirred for 1 hour. To this slurry solution was added dropwise ethyl acetate (15.3 ml) over 30 minutes, and the mixture was further stirred at 20°C for 2 hours. This slurry solution was filtered, and the obtained crystal was washed twice with ethyl acetate (25.5 ml) at 20°C. The wet crystals were dried under reduced pressure at room temperature to give (2R,3S)-3-dibenzylamino-1-chloro-2-hydroxy-4-phenylbutane sulfate as crystals (5.7 g, net. 5.6 g, 98 wt%) in a yield of 87%. As a result of HPLC analysis, it was found that peak area ratio of the compound was 99.2%, and diastereomer ratio (2R,3S)/(2S,3S) was 99.9/0.1.
melting point 117.2 to 117.4°C
¹H-NMR (methanol-d₄, 400 MHz); δppm 2.97 (dd, J=12.6, 3.2Hz, 1H), 3.12 (dd, J=14.6, 9.2Hz, 1H), 3.44 (dd, J=12.6, 3.2Hz, 1H), 3.61 (dd, J=14.6, 4.6Hz, 1H), 3.86 (ddd, J=9.2, 9.2, 4.6Hz, 1H), 4.37 (ddd, J=9.2, 3.2, 3.2Hz, 1H), 4.98 (bs, 4H), 7.12-7.17 (m, 2H), 7.27-7.35 (m, 5H), 7.40-7.48 (m, 3H), 7.50-7.58 (m, 3H), 7.64-7.72 (m, 2H)
¹³C-NMR (methanol-d₄,100 MHz); δppm 32.1, 48.5, 56.9, 64.4, 69.8, 129.3, 130.7, 130.7, 131.1,131.6,131.8,132.7,137.0
mass spectrum; m/z = 380.4 ((M-H₂SO₄)+H)⁺
[α]_{D}²⁰ = 6.0°C (c = 1.0, methanol)

### Example 2. Production of crystal of (2R,3S)-3-dibenzylamino-1-chloro-2-hydroxy-4-phenylbutane sulfate.

### Step (2c): (2R,3S)-3-dibenzylamino-1-chloro-2-hydroxy-4-phenylbutane (containing (2S,3S)-form thereof as diastereomer).

The crystal (5.0 g, 13.2 mmol, 100 wt%) of (3S)-3-dibenzylamino-1-chloro-2-oxo-4-phenylbutane obtained in the same manner as in Example 1, step (1b), was dissolved in a mixed solvent of methanol (40 ml) and dichloromethane (15 ml) under argon atmosphere, and the mixture was cooled to -10°C. To this solution was added, over 10 minutes, sodium borohydride (300 mg) divided into 10 portions, and the mixture was stirred for 1 hour. 36% Concentrated hydrochloric acid (0.682 ml) was added to the reaction solution to quench the reaction. Water (15 ml) was added at 20°C to partition the solution, and the obtained organic layer was partitioned between water (15 ml) and dichloromethane (10 ml).

### Step (2d): Production of (2R,3S)-3-dibenzylamino-1-chloro-2-hydroxy-4-phenylbutane sulfate (containing (2S,3S)-form thereof as diastereomer).

The organic layer obtained in the above-mentioned step (2c) was washed with saturated brine (15 ml), sulfuric acid (0.721 ml) was added at 20°C to give an organic layer containing (2R,3S)-3-dibenzylamino-1-chloro-2-hydroxy-4-phenylbutane sulfate containing its diastereomer ((2S,3S)-form) as an impurity.

### Step (2e): Production of crystal of (2R,3S)-3-dibenzylamino-1-chloro-2-hydroxy-4-phenylbutane sulfate.

To the organic layer obtained in the above-mentioned step (2d) was added isopropyl acetate (7.5 ml), further added a seed crystal (10 mg) of (2R,3S)-3-dibenzylamino-1-chloro-2-hydroxy-4-phenylbutane sulfate, and the mixture was stirred for 1 hour. To this slurry solution were added a mixed solvent of dichloromethane (5 ml) and isopropyl acetate (2.5 ml), further added dropwise isopropyl acetate (20 ml) over 30 min, and the mixture was stirred for 2.5 hour. This slurry solution was filtered, and the obtained crystals were washed with a mixed solvent of isopropyl acetate (7.5 ml) and dichloromethane (7.5 ml) at 20°C. The wet crystals were dried under reduced pressure at room temperature to give the object (2R,3S)-3-dibenzylamino-1-chloro-2-hydroxy-4-phenylbutane sulfate as crystal (5.3 g, yield 76%). As a result of HPLC analysis, it was found that the peak area ratio of the compound was 98.2%, and the diastereomer ratio (2R,3S)/(2S,3S) was 99.0/1.0.
melting point 102 to 105°C
mass spectrum, m/z = 380.4((M-H₂SO₄)+H)⁺
[α]_{D}²⁰= 9.2°C (c = 1.0, methanol)

### Example 3. Production of (2R,3S)-3-tert-butoxycarbonylamino-1-chloro-2-hydroxy-4-phenylbutane.

### Step (3f): Production of (2R,3S)-3-amino-1-chloro-2-hydroxy-4-phenylbutane sulfate

The crystal (5.0 g) of (2R,3S)-3-dibenzylamino-1-chloro-2-hydroxy-4-phenylbutane sulfate obtained in Example 2 was dissolved in methanol (25 ml) and, after nitrogen substitution of the reaction atmosphere, 20% palladium carbon hydroxide (50 wt% wet, 150 mg) was added to the mixture. After hydrogen substitution of the reaction atmosphere, the mixture was stirred at 40°C, and debenzylation reaction completed in 3 hours. After nitrogen substitution of the reaction atmosphere, the catalyst was filtered off, and the filtrate was partitioned between dichloromethane (15 ml) and water (15 ml). The organic layer was extracted with water (10 ml), and mixed with the aqueous layer obtained earlier. The aqueous layer was analyzed by HPLC. As a result, (2R,3S)-3-amino-1-chloro-2-hydroxy-4-phenylbutane sulfate (3.1 g) (yield 100%) was contained, and the peak area ratio was 84.5%. In addition, benzyl alcohol was contained by 8% (peak area ratio) in the aqueous layer, and other byproducts were contained by not more than 1% (peak area ratio) therein. The obtained aqueous layer was applied to the next step (3 g) without purification.

### Step (3g): Production of (2R,3S)-3-tert-butoxycarbonylamino-1-chloro-2-hydroxy-4-phenylbutane.

To the aqueous solution obtained in the above-mentioned step (3f) was added dichloromethane (10 ml), and 29% aqueous sodium hydroxide solution was further added to adjust the mixture to pH 7. Di-tert-butyldicarbonate (2.75 g) was added to the mixture, and the mixture was stirred at 25°C for 2 hours. Stirring was stopped, the mixture was partitioned, and the organic layer was washed with water (10 ml). Dichloromethane was evaporated under reduced pressure, and methanol (17 ml) and water (4.4 ml) were added to the organic layer. The mixture was cooled to 0°C, and a seed crystal of (2R,3S)-3-tert-butoxycarbonylamino-1-chloro-2-hydroxy-4-phenylbutane was added. After stirring the organic layer at 0°C for 1 hour, water (16.3 ml) was added dropwise over 30 minutes, and the mixture was further stirred for 1 hour. The slurry solution was filtered, and wet crystals were washed with a mixed solvent of ethyl acetate (0.2 ml) and heptane (10 ml). The obtained wet crystals were dried under reduced pressure at room temperature to give the object (2R,3S)-3-tert-butoxycarbonylamino-1-chloro-2-hydroxy-4-phenylbutane as white crystals (2.99 g, 100 wt%, yield 95.3%). As a result of HPLC analysis, it was found that the peak area ratio of the compound was 99.7%, and its diastereomer ((2S,3S)-form) was not detected.
¹H-NMR (CDCl₃, 300 MHz); δppm 1.38 (s, 9H), 2.91 (dd, J=8.1, 13.2Hz, 1H), 3.01 (dd, J=7.1, 13.2Hz, 1H), 3.14 (d, J=4.0Hz, 1H), 3.53 (s, 1H), 3.55 (d, J=2.3Hz, 1H), 3.70-3.77 (m, 1H), 3.79-3.89 (m, 1H), 4.88 (bd, 1H), 7.19-7.35 (m, 5H)
mass spectrum m/e; 322 (M+Na⁺)
[α]D²⁰ = -28.3° (c = 0.50, CH₂Cl₂)

### Example 4. Production of (2R,3S)-3-tert-butoxycarbonylamino-1,2-epoxy-4-phenylbutane.

### Step (4h): Production of (2R,3S)-3-tert-butoxycarbonylamino-1,2-epoxy-4-phenylbutane.

To (2R,3S)-3-tert-butoxycarbonylamino-1-chloro-2-hydroxy-4-phenylbutane (45.1 g) obtained in Example 3 were added isopropanol (120 ml) and water (45 ml), and the mixture was cooled to 0°C. 29%. Aqueous sodium hydroxide solution was added, and the mixture was stirred for 4 hours. Aqueous citric acid solution (a mixed solution of citric acid (6.73 g) and water (14 ml)) was added to the reaction mixture, and acetone (35 ml) and water (59.5 ml) were further added. A seed crystal of (2R,3S)-3-tert-butoxycarbonylamino-1,2-epoxy-4-phenylbutane was added, and this mixture was stirred for 1 hour. Water (200 ml) was added dropwise to the mixture over 1 hour, and the mixture was stirred overnight. The slurry solution was filtered, and the crystals were washed twice with aqueous acetone solution (a mixed solution of acetone (50 ml) and water (350 ml)). Wet crystals were dried under reduced pressure at room temperature to give (2R,3S)-3-tert-butoxycarbonylamino-1,2-epoxy-4-phenylbutane as white crystals (37.1 g, 100 wt%, yield 93%). As a result of HPLC analysis, it was found that the peak area ratio of the compound was 99.9%, and the diastereomer ((2S,3S)-form) was not detected.

### Comparative Example 1. Production of (2R,3S)-3-amino-1-chloro-2-hydroxy-4-phenylbutane hydrochloride.

### Step (1'a): Production of (3S)-3-dibenzylamino-1-chloro-2-oxo-4-phenylbutane (containing byproduct).

To a hexane solution (243 g, net. 176 g, 404 mmol) of N,N-dibenzyl-L-phenylalanine benzyl ester were added hexane (322 ml), tetrahydrofuran (500 ml) and bromochloromethane (44.8 ml), and the mixture was cooled to -70°C under argon atmosphere. 1.59 M n-butyllithiumhexane solution (434 ml) was added dropwise over 3.5 hours to the mixture. After stirring the reaction mixture at the same temperature for 55 minutes, the reaction mixture was added at once to saturated aqueous ammonium chloride solution (345 ml) to quench the reaction, the organic layer was separated. As a result of HPLC analysis, it was found that the organic layer contained the object compound, (3S)-3-dibenzylamino-1-chloro-2-oxo-4-phenylbutane (138 g, yield 90%), and the peak area ratio was 60.5%. In addition, the total peak area ratio of impurity was 39.5%.

### Step (1'c): Production of (2R,3S)-3-dibenzylamino-1-chloro-2-hydroxy-4-phenylbutane hydrochloride (containing (2S,3S)-form thereof as diastereomer and byproduct).

Under reduced pressure, the solvent was evaporated from the organic layer obtained in the above-mentioned step (1'a), methanol (780 ml) was added to the residue and the mixture was cooled to -10°C. Under argon atmosphere, sodium borohydride (13.0 g) divided into 10 portions was added over 10 minutes, and the mixture was stirred for 1 hour. 2 M hydrochloric acid (345 ml) was added to the reaction solution to quench the reaction to give a reaction mixture containing (2R,3S)-3-dibenzylamino-1-chloro-2-hydroxy-4-phenylbutane hydrochloride, its diastereomer ((2S,3S)-form) as impurity and a byproduct.

### Comparative Example 2. Production of (2R,3S)-3-tert-butoxycarbonylamino-1-chloro-2-hydroxy-4-phenylbutane (containing (2S,3S)-form thereof as diastereomer and byproduct).

### Step (2'f): Production of (2R,3S)-3-amino-1-chloro-2-hydroxy-4-phenylbutanehydrochloride (containing (2S,3S)-form thereof as diastereomer and byproduct).

The reaction mixture containing (2R,3S)-3-dibenzylamino-1-chloro-2-hydroxy-4-phenylbutane hydrochloride obtained in Comparative Example 1 (1'c) was subjected to nitrogen substitution, and 20% palladium carbon hydroxide (50 wt% wet, 9.8 g) was added. After hydrogen substitution of the reaction atmosphere, the mixture was stirred at 40°C, and debenzylation reaction completed in 22 hours. After nitrogen substitution of the reaction atmosphere, palladium carbon hydroxide was filtered off. The filtrate was concentrated under reduced pressure, and 2M hydrochloric acid (195 ml) was added to the residue. The mixture was concentrated again and the obtained residue was partitioned between dichloromethane (225 ml) and water (75 ml). The obtained aqueous layer was analyzed by HPLC. As a result, (2R,3S)-3-amino-1-chloro-2-hydroxy-4-phenylbutane hydrochloride and its (2S,3S)-form as a diastereomer thereof (75.6 g, yield 88%) were contained, and the total peak area ratio was 75.0%. In addition, the aqueous layer contained benzyl alcohol (8%, peak area ratio), and other byproduct (7.5%, peak area ratio).

### Step (2'g): Production of (2R,3S)-3-tert-butoxycarbonylamino-1-chloro-2-hydroxy-4-phenylbutane (containing (2S,3S)-form thereof as diastereomer).

To an aqueous solution of (2R,3S)-3-amino-1-chloro-2-hydroxy-4-phenylbutane hydrochloride (74.4 g) containing its (2S,3S)-form as a diastereomer thereof, which had been obtained in the above-mentioned step (2'f), was added toluene (378 ml), and the mixture was vigorously stirred. The mixture was adjusted to pH 7 with 4 M aqueous sodium hydroxide solution. Keeping pH 7 with 4 M aqueous sodium hydroxide solution, the mixture was added to a mixed solution of di-tert-butyldicarbonate (68.7 g) and toluene (94 ml), and the mixture was vigorously stirred at 25°C for 3 hours. The organic layer (606.4 g) was separated, cooled to 0°C and filtered. As a result of HPLC analysis, it was found that the filtrate contained (2R,3S)-3-tert-butoxycarbonylamino-1-chloro-2-hydroxy-4-phenylbutane (64.9 g, yield 68%), and the diastereomer ratio (2R,3S)/(2S,3S) was 95/5. In addition, the peak area ratio of other byproduct was 19.5% relative to (2R,3S)-3-tert-butoxycarbonylamino-1-chloro-2-hydroxy-4-phenylbutane.

### Comparative Example 3. Production of (2R,3S)-3-tert-butoxycarbonylamino-1,2-epoxy-4-phenylbutane (containing (2S,3S)-form as diastereomer thereof).

### Step (3'h): Production of (2R,3S)-3-tert-butoxycarbonylamino-1,2-epoxy-4-phenylbutane (containing (2S,3S)-form as diastereomer thereof).

The organic layer (26.5 g) obtained in Comparative Example 2, step (2'g), was concentrated under reduced pressure, isopropanol (2 ml) was added to the residue, and the mixture was concentrated again to dryness. Isopropanol (21.8 ml) and water (3.0 ml) were added to the residue and the mixture was cooled to 0°C. Then, 6 M aqueous sodium hydroxide solution (2.7 ml) and water (1.2 ml) were added to the solution, and the mixture was reacted for 8.5 hours. An aqueous solution (36.6 ml) of citric acid (351 mg) was added to the reaction mixture, and the mixture was cooled from 0°C to -10°C over 3 hours. A seed crystal of (2R,3S)-3-tert-butoxycarbonylamino-1,2-epoxy-4-phenylbutane was added, and the mixture was stirred at -10°C for 3 days and filtered. The obtained crystals were dried under reduced pressure to give adhesive orange crystals (2.47 g). As a result of HPLC analysis, it was found that the content of the object compound, (2R,3S)-3-tert-butoxycarbonylamino-1,2-epoxy-4-phenylbutane, was 2.07 g, 83.8 wt%, and the yield was 83%. In addition, the crystals contained 0.071 g of a diastereomer ((2S,3S)-form)), and the diastereomer ratio (2R,3S)/(2S,3S) was 96.7/3.3. Moreover, the peak area ratio of other byproduct was 10% relative to the object compound, (2R,3S)-3-tert-butoxycarbonylamino-1,2-epoxy-4-phenylbutane.

### Example 5. Production of crystals of various salts of (2R,3S)-3-dibenzylamino-1-chloro-2-hydroxy-4-phenylbutane.

In view of the fact that the purification of compound (3) by crystal precipitation is conventionally difficult, the diastereomer ratio of compound (3) depends on the reduction selectivity of compound (2). Therefore, by reduction with sodium borohydride, which is applicable to industrial production, the ratio of (2R,3S)- or (2S,3R)-3-dibenzylamino-1-chloro-2-hydroxy-4-phenylbutane to its diastereomer is about 95:5 at most. In addition, WO96/17821 reports crystal precipitation of (2S,3S)-3-dibenzylamino-1-chloro-2-hydroxy-4-phenylbutane hydrochloride from a methanol solution.

Then, the present inventors have tried crystal precipitation of hydrochloride of compound (3), which is a (2S,3S)-form diastereomer, from methanol or ethyl acetate, but crystal precipitation did not occur and the residue obtained by evaporation of the solvent was an oily substance, thus failing to obtain crystals.

Accordingly, the present inventors conducted the following experiment in an attempt to find the combination of solvent and acid, which is suitable for the crystallization of compound (3).

The reaction mixture containing (2R,3S)-3-dibenzylamino-1-chloro-2-hydroxy-4-phenylbutane hydrochloride, which was obtained in Comparative Example 1, (1'c), (11 g in total of (2R,3S)-3-dibenzylamino-1-chloro-2-hydroxy-4-phenylbutane hydrochloride and (2S,3S)-form as its diastereomer, (2S,3S)/(2R,3S) = 11/89) was concentrated, dichloromethane (28 ml) and water (28 ml) were added, and the mixture was adjusted to pH 7.0 with 15% aqueous potassium carbonate solution. The mixture was partitioned between the organic layer and the aqueous layer, the obtained organic layer was concentrated to dryness to give an oil. The oil was divided and various combinations of the following solvents (0.25 ml/g) and acids (1 molar equivalent) were added at room temperature, the mixtures were stirred, and the solvents were evaporated to see if a salt with each acid could be precipitated as crystals.

Solvents used: methanol, ethanol, isopropanol, ethyl acetate, isopropyl acetate, and acetonitrile

Acids used: oxalic acid, malonic acid, succinic acid, fumaric acid, phthalic acid, citric acid, glycol acid, (-)(D)tartaric acid, (+)(L)tartaric acid, (L)(-)dibenzoyltartaric acid, (D)(+)dibenzoyltartaric acid, malic acid, methanesulfonic acid, tosyl acid, (+)-10-camphorsulfonic acid, (-)-10-camphorsulfonic acid, aspartic acid, glutamic acid, acetic acid, phosphoric acid, trifluoroacetic acid, and sulfuric acid.

From the above-mentioned experiment, it was found that when ethyl acetate or isopropyl acetate was used as a solvent and sulfuric acid was used as an acid, (2R,3S)-3-dibenzylamino-1-chloro-2-hydroxy-4-phenylbutane sulfate could be precipitated as crystals (crystal of compound (4-a)). In contrast, when other combinations of acids and solvents were used, (2R,3S)-3-dibenzylamino-1-chloro-2-hydroxy-4-phenylbutane sulfate did not precipitate as crystals.

Moreover, it was also found that when a slurry of the above-mentioned crystal obtained using sulfuric acid and ethyl acetate or isopropyl acetate was filtered, the diastereomer ((2S,3S)-form) contained in the reaction mixture obtained in Example 1, (1c), was mostly transferred to the mother liquor, leaving no diastereomer in the crystals thus obtained.

## Claims

1. A method of producing crystals of 3-dibenzylamino-1-chloro-2-hydroxy-4-phenylbutane sulfate represented by formula (4): 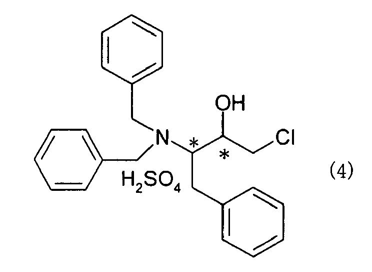 wherein * means an asymmetric carbon atom, and the configuration at the 2-position and the 3-position is (2R,3S) when the configuration of the following formula (3) is (2R,3S), or (2S,3R) when the configuration of the following formula (3) is (2S,3R),
said method comprising:
(a) reacting lithiumchloromethane with a N,N-dibenzylphenylalanine ester represented by formula (1): 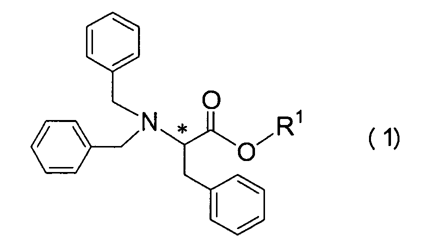 wherein R¹ is an alkyl group having 1 to 10 carbon atoms, an aryl group having 6 to 15 carbon atoms or an aralkyl group having 7 to 20 carbon atoms, each of which optionally having one or more substituents, or any of these groups containing one or more heteroatoms in a carbon skeleton, * means an asymmetric carbon atom, and the configuration at the 2-position is S or R, to obtain 3-dibenzylamino-1-chloro-2-oxo-4-phenylbutane represented by formula (2): 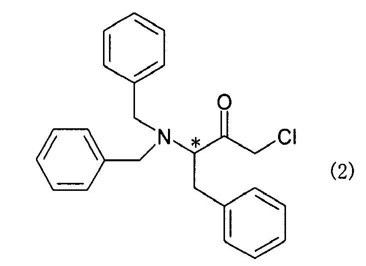 wherein * means an asymmetric carbon atom, the configuration at the 3-position is S when the configuration at the 2-position of the compound of formula (1) is S, or R when the configuration at the 2-position of the compound of formula (1) is R, and at least one byproduct;
(b) dissolving said 3-dibenzylamino-1-chloro-2-oxo-4-phenylbutane represented by formula (2) and the byproduct in a polar solvent and adding water to the solution to precipitate crystals of said 3-dibenzylamino-1-chloro-2-oxo-4-phenylbutane represented by formula (2);
(c) reducing said crystals of 3-dibenzylamino-1-chloro-2-oxo-4-phenylbutane represented by formula (2) to obtain 3-dibenzylamino-1-chloro-2-hydroxy-4-phenylbutane represented by formula (3): 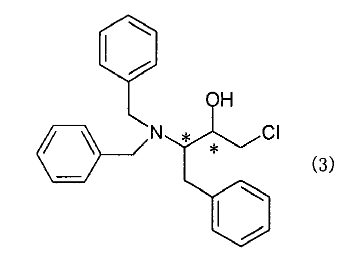 wherein * means an asymmetric carbon atom, the configuration at the 2-position and the 3-position is (2R,3S) when the configuration at the 3-position of the compound of formula (2) is S, or (2S,3R) when the configuration at the 3-position of the compound of formula (2) is R, and at least its diastereomer as an impurity;
(d) adding sulfuric acid to the resulting 3-dibenzylamino-1-chloro-2-hydroxy-4-phenylbutane represented by formula (3) and at least its diastereomer as an impurity to obtain 3-dibenzylamino-1-chloro-2-hydroxy-4-phenylbutane sulfate represented by formula (4) and at least its diastereomer as an impurity; and
(e) precipitating crystals of said 3-dibenzylamino-1-chloro-2-hydroxy-4-phenylbutane sulfate represented by formula (4) from a solution containing acetic acid ester or acetic acid ester.

2. A method of producing a 3-protected amino-1-chloro-2-hydroxy-4-phenylbutane represented by formula (6): 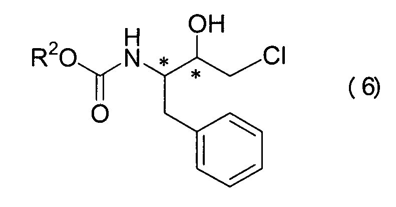 wherein R² is an alkyl group having 1 to 10 carbon atoms or an aralkyl group having 7 to 20 carbon atoms, * means an asymmetric carbon atom, the configuration at the 2-position and the 3-position is (2R,3S) when the configuration of the compound of formula (5) below is (2R,3S), or (2S,3R) when the configuration of the compound of formula (5) below is (2S,3R),
said method comprising:
(f) catalytically reducing the crystal of 3-dibenzylamino-1-chloro-2-hydroxy-4-phenylbutane sulfate represented by formula (4), which is obtained according to the method of claim 1, to obtain 3-amino-1-chloro-2-hydroxy-4-phenylbutane sulfate represented by formula (5): 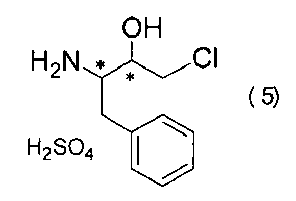 wherein * means an asymmetric carbon atom, the configuration at the 2-position and the 3-position is (2R,3S) when the configuration of the compound of formula (4) is (2R,3S), or (2S,3R) when the configuration of the compound of formula (4) is (2S,3R); and
(g) protecting the amino group of said 3-amino-1-chloro-2-hydroxy-4-phenylbutane sulfate represented by formula (5) with a protecting agent under a neutral condition.

3. A method of producing a 3-protected amino-1,2-epoxy-4-phenylbutane represented by formula (7): 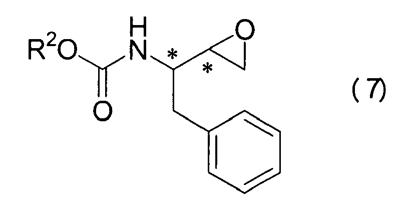 wherein R² is an alkyl group having 1 to 10 carbon atoms or an aralkyl group having 7 to 20 carbon atoms, * means an asymmetric carbon atom, and the configuration at the 2-position and the 3-position is (2R,3S) when the configuration of the compound of formula (6) is (2R,3S), or (2S,3R) when the configuration of the compound of formula (6) is (2S,3R),
said method comprising:
(h) treating a 3-protected amino-1-chloro-2-hydroxy-4-phenylbutane represented by formula (6), which is obtained according to the method of claim 2, with a base.

4. A method of producing crystals of 3-dibenzylamino-1-chloro-2-hydroxy-4-phenylbutane sulfate represented by formula (4): 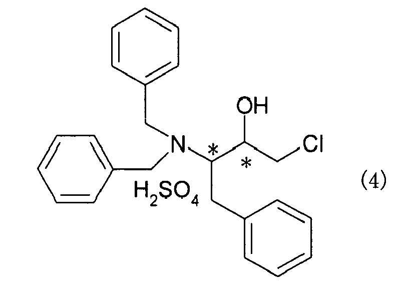 wherein * means an asymmetric carbon atom, the configuration at the 2-position and the 3-position is (2R,3S) when the configuration of the following formula (3) is (2R,3S), or (2S,3R) when said configuration the configuration of the following formula (3) is (2S,3R),
said method comprising:
(d) adding sulfuric acid to 3-dibenzylamino-1-chloro-2-hydroxy-4-phenylbutane represented by formula (3): 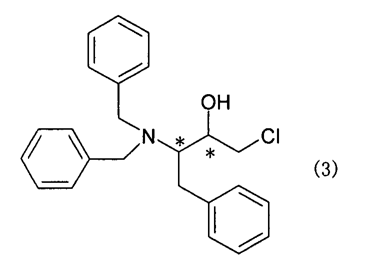 wherein * means an asymmetric carbon atom, and the configuration at the 2-position and the 3-position is (2R,3S) or (2S,3R), to obtain 3-dibenzylamino-1-chloro-2-hydroxy-4-phenylbutane sulfate represented by formula (4) and at least its diastereomer as an impurity; and
(e) precipitating crystals of said 3-dibenzylamino-1-chloro-2-hydroxy-4-phenylbutane sulfate represented by formula (4) from a solution containing acetic acid ester or acetic acid ester.

5. A method of producing crystals of (2R,3S)-3-dibenzylamino-1-chloro-2-hydroxy-4-phenylbutane sulfate represented by formula (4-a): 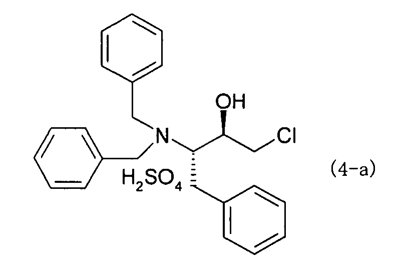 said method comprising:
(aa) reacting lithiumchloromethane with (S)-N,N-dibenzylphenylalanine ester represented by formula (1-a): 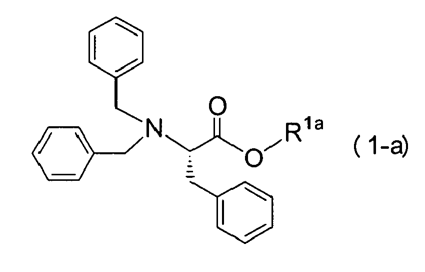 wherein R^{1a} is an alkyl group having 1 to 10 carbon atoms, an aryl group having 6 to 15 carbon atoms or an aralkyl group having 7 to 20 carbon atoms, each of which optionally having one or more substituents, or any of these groups containing one or more heteroatoms in a carbon skeleton, to obtain (3S)-3-dibenzylamino-1-chloro-2-oxo-4-phenylbutane represented by formula (2-a): 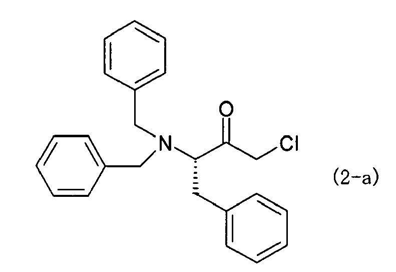 and at least a byproduct;
(ab) dissolving said (3S)-3-dibenzylamino-1-chloro-2-oxo-4-phenylbutane represented by formula (2-a) and the byproduct in a polar solvent and adding water to the solution to precipitate crystals of said (3S)-3-dibenzylamino-1-chloro-2-oxo-4-phenylbutane represented by formula (2-a);
(ac) reducing said crystals of (3S)-3-dibenzylamino-1-chloro-2-oxo-4-phenylbutane represented by formula (2-a) to obtain (2R,3S)-3-dibenzylamino-1-chloro-2-hydroxy-4-phenylbutane represented by formula (3-a): 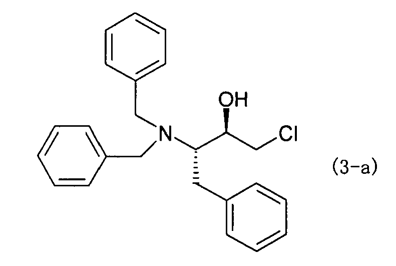 and at least its diastereomer as an impurity;
(ad) adding sulfuric acid to said (2R,3S)-3-dibenzylamino-1-chloro-2-hydroxy-4-phenylbutane represented by formula (3-a) and at least its diastereomer as an impurity to obtain (2R,3S)-3-dibenzylamino-1-chloro-2-hydroxy-4-phenylbutane sulfate represented by formula (4-a) and at least its diastereomer as an impurity; and
(ae) precipitating crystals of said (2R,3S)-3-dibenzylamino-1-chloro-2-hydroxy-4-phenylbutane sulfate represented by formula (4-a) from a solution containing acetic acid ester or acetic acid ester.

6. A method of producing a (2R,3S)-3-protected amino-1-chloro-2-hydroxy-4-phenylbutane represented by formula (6-a): 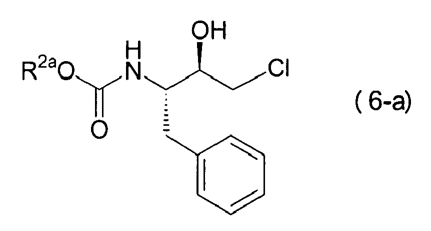 wherein R^{2a} is an alkyl group having 1 to 10 carbon atoms or an aralkyl group having 7 to 20 carbon atoms,
said method comprising:
(af) catalytically reducing crystals of said (2R,3S)-3-dibenzylamino-1-chloro-2-hydroxy-4-phenylbutane sulfate represented by formula (4-a), which are obtained according to the method of claim 5, to obtain (2R,3S)-3-amino-1-chloro-2-hydroxy-4-phenylbutane sulfate represented by formula (5-a): 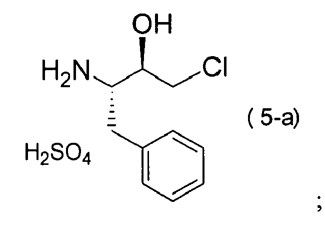 and
(ag) protecting the amino group of said (2R,3S)-3-amino-1-chloro-2-hydroxy-4-phenylbutane sulfate represented by formula (5-a) with a protecting agent under a neutral condition.

7. A method of producing a (2R,3S)-3-protected amino-1,2-epoxy-4-phenylbutane represented by formula (7-a): 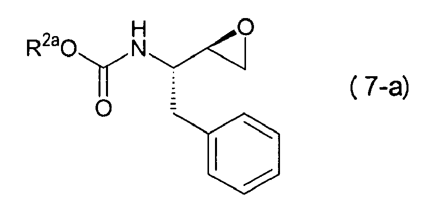 wherein R^{2a} is an alkyl group having 1 to 10 carbon atoms or an aralkyl group having 7 to 20 carbon atoms,
said method comprising:
(ah) treating said (2R,3S)-3-protected amino-1-chloro-2-hydroxy-4-phenylbutane represented by formula (6-a), which is obtained according to the method of claim 6, with a base.

8. A method of producing crystals of (2S,3R)-3-dibenzylamino-l-chloro-2-hydroxy-4-phenylbutane sulfate represented by formula (4-b): 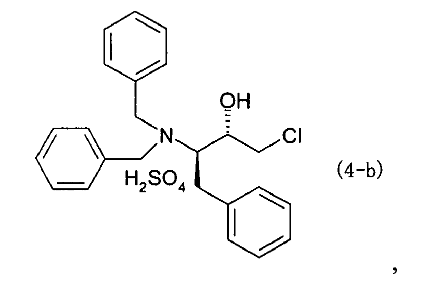 said method comprising:
(ba) reacting lithiumchloromethane with (R)-N,N-dibenzylphenylalanine ester represented by formula (1-b): 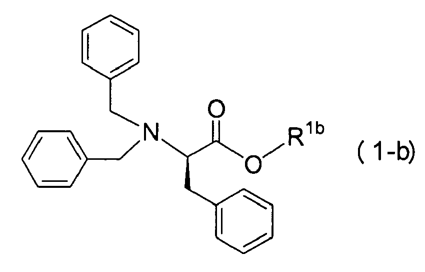 wherein R^{1b} is an alkyl group having 1 to 10 carbon atoms, an aryl group having 6 to 15 carbon atoms or an aralkyl group having 7 to 20 carbon atoms, each of which optionally having one or more substituents, or any of these groups containing one or more heteroatoms in a carbon skeleton, to obtain (3R)-3-dibenzylamino-1-chloro-2-oxo-4-phenylbutane represented by formula (2-b): 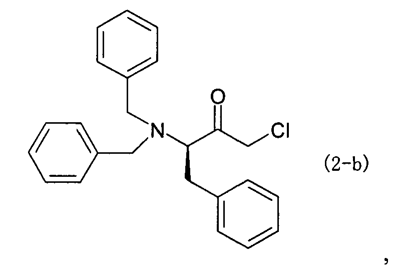 and at least one byproduct;
(bb) dissolving said (3R)-3-dibenzylamino-1-chloro-2-oxo-4-phenylbutane represented by formula (2-b) and the byproduct, in a polar solvent and adding water to the solution to precipitate crystals of said (3R)-3-dibenzylamino-1-chloro-2-oxo-4-phenylbutane represented by formula (2-b);
(bc) reducing the crystals of said (3R)-3-dibenzylamino-1-chloro-2-oxo-4-phenylbutane represented by formula (2-b) to obtain (2S,3R)-3-dibenzylamino-1-chloro-2-hydroxy-4-phenylbutane represented by formula (3-b): 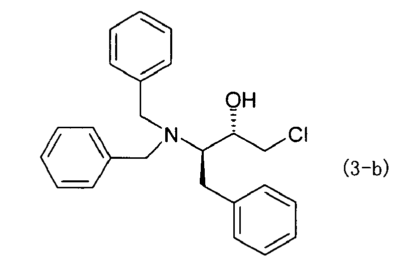 and at least its diastereomer as an impurity;
(bd) adding sulfuric acid to said (2S,3R)-3-dibenzylamino-1-chloro-2-hydroxy-4-phenylbutane represented by formula (3-b) and at least its diastereomer as an impurity to obtain (2S,3R)-3-dibenzylamino-1-chloro-2-hydroxy-4-phenylbutane sulfate represented by formula (4-b) and at least its diastereomer as an impurity; and
(be) precipitating crystals of said (2S,3R)-3-dibenzylamino-1-chloro-2-hydroxy-4-phenylbutane sulfate represented by formula (4-b) from a solution containing acetic acid ester or acetic acid ester.

9. A method of producing a (2S,3R)-3-protected amino-1-chloro-2-hydroxy-4-phenylbutane represented by formula (6-b): 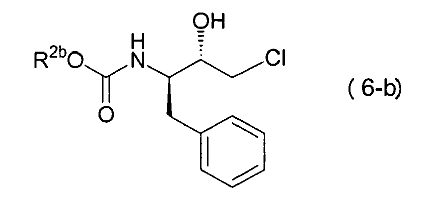 wherein R^{2b} is an alkyl group having 1 to 10 carbon atoms or an aralkyl group having 7 to 20 carbon atoms,
said method comprising:
(bf) catalytically reducing crystals of said (2S,3R)-3-dibenzylamino-1-chloro-2-hydroxy-4-phenylbutane sulfate represented by formula (4-b), which are obtained according to method of claim 8, to obtain (2S,3R)-3-amino-1-chloro-2-hydroxy-4-phenylbutane sulfate represented by formula (5-b): 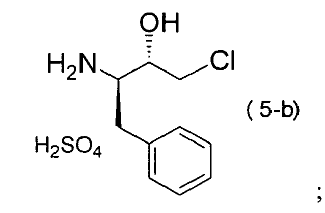 and
(bg) protecting the amino group of said (2S,3R)-3-amino-1-chloro-2-hydroxy-4-phenylbutane sulfate represented by formula (5-b) with a protecting agent under a neutral condition.

10. A method of producing a (2S,3R)-3-protected amino-1,2-epoxy-4-phenylbutane represented by formula (7-b): 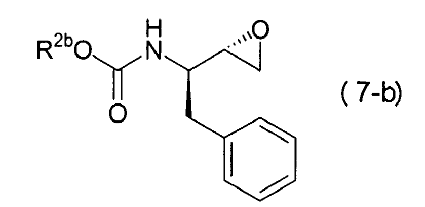 wherein R^{2a} is an alkyl group having 1 to 10 carbon atoms or an aralkyl group having 7 to 20 carbon atoms,
said method comprising:
(bh) treating said (2S,3R)-3-protected amino-1-chloro-2-hydroxy-4-phenylbutane represented by formula (6-b), which is obtained according to the method of claim 9, with a base.

11. The method of claim 2 or 3, wherein R² is a tert-butyl group, a benzyl group, or a fluorenylmethyl group.

12. The method of claim 6 or 7, wherein R^{2a} is a tert-butyl group, a benzyl group, or a fluorenylmethyl group.

13. The method of claim 9 or 10, wherein R^{2b} is a tert-butyl group, a benzyl group, or a fluorenylmethyl group.

14. The method of claim 1, wherein R¹ is a methyl group, an ethyl group, an isobutyl group, or a benzyl group.

15. The method of claim 5, wherein R^{1a} is a methyl group, an ethyl group, an isobutyl group, or a benzyl group.

16. The method of claim 8, wherein R^{1b} is a methyl group, an ethyl group, an isobutyl group, or a benzyl group.

17. The method of any one of claims 1 to 16, wherein said acetic acid ester comprises one or more members selected from the group consisting of methyl acetate, ethyl acetate, propyl acetate, and mixtures thereof.

18. 3-Dibenzylamino-1-chloro-2-hydroxy-4-phenylbutane sulfate represented by formula (4): 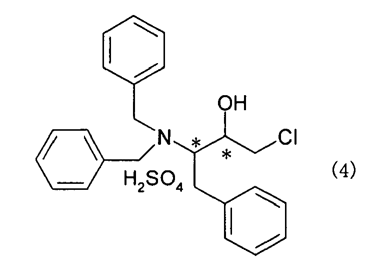 wherein * means an asymmetric carbon atom, and the configuration at the 2-position and the 3-position is (2R,3S) or (2S,3R).

19. (2R,3S)-3-Dibenzylamino-1-chloro-2-hydroxy-4-phenylbutane sulfate represented by formula (4-a):

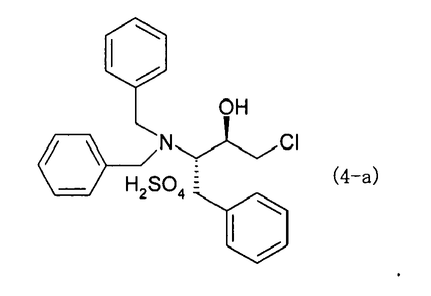

20. (2S,3R)-3-Dibenzylamino-1-chloro-2-hydroxy-4-phenylbutane sulfate represented by formula (4-b):
